# EUROPEAN PATENT SPECIFICATION

(11) **EP 4 324 504 B1**
(45) Date of publication and mention of the grant of the patent: **23.07.2025**
(21) Application number: 22724805.1
(22) Date of filing: 07.04.2022
(51) Int. Cl.: A61M 16/00, A61M 16/04, A61M 16/08, A61M 16/12, A61M 16/20, A61M 16/10, A61M 16/16

(54) **VENTILATOR FOR MECHANICAL VENTILATION**
BEATMUNGSGERÄT FÜR MECHANISCHE BEATMUNG
RESPIRATEUR POUR VENTILATION MÉCANIQUE

(30) Priority: 16.04.2021 ES 202130335
(43) Date of publication of application: 21.02.2024
(73) Proprietor: Picazo Sotos, Lucas, 11080 Cádiz (ES); Quintanilla Urionabarrenechea, Alexander, 48014 Bilbao (ES)
(72) Inventor: Picazo Sotos, Lucas, 11080 Cádiz (ES); Quintanilla Urionabarrenechea, Alexander, 48014 Bilbao (ES)
(74) Representative: Clarke, Modet y Cía., S.L.
(86) International application number: PCT/ES2022/070215
(87) International publication number: WO 2022/219215

(56) References cited:
- EP-A1- 0 234 736
- WO-A1-2022/060231
- ES-B1- 2 121 691
- US-A- 4 502 481
- US-A- 5 097 424
- US-A1- 2008 017 198

## Description

### FIELD OF THE INVENTION

This invention relates to a ventilator for invasive mechanical ventilation and associated flow control and conditioning equipment, especially designed for use in patients with acute respiratory failure on orotracheal intubation and requiring mechanical ventilation, such as patients infected by the SARS-COV2 microorganism.

### BACKGROUND ART

Acute respiratory failure is a condition in which the patient has an alteration in the capacity of the lungs to exchange oxygen and carbon dioxide with the organism. When the respiratory insufficiency is very important, it requires orotracheal intubation, which is a measure in which a pharmacological coma is induced in the patient and a tube is introduced into the trachea to connect him/her to a machine that helps him/her to breathe. This machine is known as a ventilator.

COVID-19 infection is a viral disease with a high contagious capacity, with a non-negligible morbidity rate and a very high consumption of health resources, which is causing a progressive worldwide emergency, blocking health, human, pharmaceutical and technological resources. The main complication is severe pneumonia with the development of acute respiratory distress syndrome or ARDS, requiring in a high percentage of cases orotracheal intubation and connection to mechanical ventilation. The lack of supply of ventilators due to the high demand in the current pandemic is one of the main current concerns as it leads to the saturation of the health care system by not being able to attend or treat patients who need to receive these treatment measures. In this context, there is a need for ventilators for invasive mechanical ventilation that can be distributed widely and at low cost.

Typical ventilators deliver gases (O₂ and air) through conventional tubing and have two opening and closing valves. The inspiratory valve allows air to be delivered to the lungs during inspiratory time, and the expiratory valve, when open, allows air to exit from the lung to the outside. These ventilators are complex and require precise coordination of these valves for proper operation.

Documents US4502481A, US5097424A, US2008/017198A1 and EP0234736A1 refer to devices and/or systems for mechanical ventilation.

Document ES2121691B1 shows an extratracheal continuous jet mechanical ventilation system (ET-CJV), as a time-controlled mechanical ventilation technique characterized by a continuous gas flow applied to the endotracheal tube inlet, which improves the oxygenation of treated patients allowing an effective increase in oxygen transport. This continuous flow provides positive conditions for the ventilation of patients with pathologies such as Acute Respiratory Distress Syndrome (ARDS), which presents with radiological pulmonary infiltrates, hypoxemia, decreased pulmonary compliance and increased intrapulmonary shunt. Being a constant flow, ventilation can be performed only by opening and closing a time-controlled electromagnetic valve placed in the expiratory branch.

Despite the advantages of the extratracheal continuous jet mechanical ventilation (ET-CJV) system, it should be noted that it acts as an auxiliary element of the pressure control ventilation (PCV) provided by a conventional ventilator, and not as a stand-alone ventilator.

The ventilator of this invention works based on continuous gas flow that is directed through a nozzle that accelerates and creates a turbulent flow that acts as an expiratory brake maximizing the effect of end-expiratory pressure (PEEP), thus keeping the lung more open. Unlike the mechanical ventilation system by continuous extratracheal jet (VJC-ET), the ventilator of the present invention works as an independent ventilator, which allows ventilating only with the continuous jet flow, adding safety and control elements.

The ventilator of the present invention presents an optimized and innovative design, especially in the joint use of continuous flow, flow control and conditioning equipment with nozzle and all the technification, both of operation and monitoring of the therapy offered to the patient, to adapt to the current regulatory measures both of treatment and safety.

It is a simple and economical ventilator that allows, in a safe and effective way, to ventilate and oxygenate patients with severe respiratory failure. With only one valve, it simplifies the ventilator mechanism without the need for valve coordination of classic ventilators. In addition, it presents a simplified handling in such a way that the user only must set the inflow to the ventilator, the desired inspired oxygen fraction, the inspiratory volume, the inspiratory time, and the respiratory frequency to be offered to the patient.

### SUMMARY OF INVENTION

The ventilator for mechanical ventilation of the present invention is defined by the features of claim 1. Preferred embodiments of the same are defined by the features of the dependent claims 2-13.

The continuous flow ventilator for mechanical ventilation of the present invention comprises a control box, an inspiratory limb configured to receive an air-oxygen mixture, an expiratory limb, and an expiratory valve disposed in the expiratory limb.

The ventilator comprises flow control and conditioning equipment to which the inspiratory limb and the expiratory limb are connected, configured to deliver a continuous flow of said air-oxygen mixture to a patient through an endotracheal tube, where said continuous flow is kept constant during inspiration and expiration of the patient, so that when the expiratory valve opens, there is a collision of the continuous flow with the expiratory flow coming from the patient, causing an expiratory brake and an intrinsic PEEP end-expiratory pressure.

Thus, the ventilator is essentially characterized by the continuous presence of a gas flow (mixture of air and oxygen) entering the system. During the inspiration phase, this flow is directed into the patient's respiratory system via an endotracheal tube. During the expiratory phase, this flow is evacuated from the system after having circulated through the ventilator, which confers the clinical advantages that characterize this device.

When the expiratory valve opens, the system configuration causes the expiratory flow to collide with the continuous flow caused by the flow control and conditioning equipment. Specifically, the elastic retraction of the thorax allows the passive exit of part of the intrathoracic air, due to the difference in intrapulmonary pressure and that generated by the continuous flow. A current of exhaled gas is produced against the continuous flow, maintaining a counterpressure during the entire expiratory time. This produces two effects: an intrinsic PEEP effect of the system and an expiratory brake effect, which prevents a sudden drop in pressure in the expiratory phase. In addition, this expiratory brake limits the shear forces due to the sudden pressure drop in the collapsed and ventilated zones, minimizing the risk of barotrauma. Being a constant flow system, the inspiratory volume (Vt of tidal volume) is provided by the product of the instantaneous gas flow by the inspiratory time (time in which the expiratory valve is closed). The expiratory valve is preferably a solenoid valve or solenoid valve with single open or closed position controlled by the central control equipment.

The intrinsic PEEP produced by the ventilator depends mainly on the total gas flow, the configuration of the flow control and conditioning equipment (FACT), the expiratory time and the thoraco-pulmonary distensibility.

According to a first configuration of the flow control and conditioning equipment, called ECAF PRO, it produces an intrinsic PEEP of the system between 6 and 11 cm H2O for flows between 20 I/min and 30 I/min respectively.

According to a second configuration of the flow control and conditioning equipment, referred to as ECAF STANDARD, it produces a system intrinsic PEEP of between 2 and 3 cm H2O for flow rates of between 20 I/min and 30 I/min respectively.

Preferably, the ventilator comprises a PEEP valve arranged in series with the expiratory valve on the expiratory limb, configured to cause a pressure at end-expiratory PEEP added.

The added PEEP is in addition to the intrinsic PEEP of the system itself. Thus, the total PEEP of the system is regulated according to medical judgment, considering the intrinsic PEEP, and adjusting the PEEP valve until the desired total PEEP is reached. Preferably, the PEEP valve can provide an added PEEP of between 0 to 22 cm H₂O. The adjustment is made by continuously monitoring the total PEEP by displaying the same for each respiratory cycle on the control panel display.

Preferably, the PEEP valve is placed downstream of the expiratory valve at an added pressure port on the control panel, although it could also be placed at any other point on the device upstream of the expiratory valve.

The total PEEP of the system can be instantly monitored and displayed on the control panel, preferably by means of a pressure-time graph.

The patient treated with invasive mechanical ventilation is very susceptible to infection, and dirty or contaminated equipment is a potential source of infection. To reduce such risks, the ventilator and its accessories should be cleaned regularly and systematically before and after each use.

Also, to further minimize the risk of infection, preferably, the ventilator comprises an antibacterial and/or antiviral filter arranged in series with the expiratory valve on the expiratory limb. The maximum pressure loss generated by the filter should be up to 2 cm H₂O for a flow rate of 30 litters/minute. The filtration efficiency must be greater than 99% and the compressible volume of 42 ml, for a minimum tidal volume of 150 ml.

Preferably, the ventilator comprises an adjustable safety relief valve connected to the flow control and conditioning equipment configured to limit the inspiratory branch pressure. It is thus a relief valve configured to prevent overpressure in the system. The flow resistance is preferably less than 1 cm H₂O, for the range of working flow rates. The relief pressure of the adjustable safety relief valve is adjustable from 0 to 60 cm H₂O. The system preferably operates with the valve set at 35 cm H₂O under the specified conditions. In case of overpressure, an audible and/or visual signal is generated on the control panel and the system is brought to the safety position.

Preferably, the ventilator comprises a pressure transmitter connected to the flow control and conditioning equipment configured to monitor the pressure during the breathing cycle. Said transmitter preferably operates on a scale between 0 and 100 cm H₂O. It captures the system pressure through a pressure tap connected to a pressure transmitter in the control panel. Through this system the pressure during the respiratory cycle is monitored and displayed on a pressure-time graph, as well as the peak pressure and total PEEP values for each respiratory cycle. All these parameters can be viewed on the display on the control panel screen.

Preferably, the ventilator comprises an oxygen sensor in the inspiratory limb, configured to measure the inspired fraction of oxygen FiO₂. Said measured inspired fraction of oxygen (measured FiO₂), should not vary by more than ±3% over the desired FiO₂. A higher percentage could be the result of an error in the flow meter flow setting. The oxygen sensor emits a signal proportional to the amount of oxygen detected in the fluid injected to the patient. When the inspired oxygen fraction FiO₂ is outside this range, an alarm signal on the control panel warns that the ventilator is to be operated regardless of the oxygen sensor measurement.

Preferably, the ventilator comprises an active humidifier in the inspiratory branch, configured to humidify the air-oxygen mixture. Said active humidifier may be of the cascade humidification or bubble humidification type.

Preferably, the ventilator comprises a gas inlet port configured to receive the air-oxygen mixture coming from a mixer. This gas inlet port is preferably a 9/16" UNF inlet with a hose nipple according to the applicable standard, arranged on the control panel, to which a common oxygen line of 6 mm outside diameter, available in any hospital, relates to the selected gas mixture. The maximum inlet pressure to the system is preferably 600 kPa and the maximum total inlet flow rate is 45 litters/minute. The gases admitted by the system are compressed air and oxygen.

The mixer allows the inspired fraction of oxygen (FiO₂) to be regulated by the mixer and connected by means of the extension to the control panel inlet. In case you want to give a FiO₂ of 1, only an extension cord is connected from the O₂ flowmeter to the control panel input.

The mixer can use an adjustable oxygen flowmeter up to at least 30 litters/minute, and an adjustable compressed air flowmeter of 15 I/min or 30 I/min, depending on the ventilation conditions.

The gas inlet port may additionally include a "Y" connection for separate connection of air and oxygen. If a FiO₂ other than 1 is desired and a mixer is not available, this connection can be used to mix the oxygen and compressed air flows in the amount indicated on the control panel when the desired tidal volume and FiO2 are entered.

The functions of the control panel are mainly: powering the system, collecting, and interpreting the collected data, displaying and configuring the ventilation parameters, displaying and configuring the set alarms, executing the set alarms, executing the programmed safety criteria, as well as containing part of the system components.

Preferably, the front of the control panel includes a manual emergency button that allows the manual opening of the expiratory valve for patient safety.

The main alarms of the system are: power failure, pressure stabilization for 10 seconds (variations below 5 cm H₂0), low pressure, minimum PEEP, maximum PEEP, peak pressure of the cycle above the set pressure, overpressure alarm, FiO₂ out of range, solenoid valve maintenance warning and activation of the emergency button.

The flow control and conditioning equipment, also called ECAF, presents a tubular configuration comprising a plurality of pneumatically communicated connections. Preferably, the flow control and conditioning equipment is made of polyamide.

The flow control and conditioning equipment comprises:
- an inspiratory inlet port configured to connect to the inspiratory limb to receive the air-oxygen mixture;
- an endotracheal outlet port, aligned with the inspiratory inlet port, configured to connect to the endotracheal tube and deliver a continuous flow of the air-oxygen mixture to the patient; and
- an expiratory outlet port, arranged in a "T" between the inspiratory inlet port and the endotracheal outlet port, configured to connect to the expiratory limb and allow expiratory flow from the patient to exit.

Preferably, the inspiratory inlet connection is a 22 mm diameter female connection, for the connection of a 22 mm corrugated tube coming from an inspiratory outlet arranged in the control panel, or from the humidifier, if available.

Preferably, the endotracheal outlet connection is a 15 mm diameter female connection for connection of the endotracheal tube.

Preferably, the expiratory outlet connection is a 22 mm diameter female connection, for connection of a 22 mm corrugated tube to an expiratory inlet arranged on the control panel.

Preferably, the flow control and conditioning equipment comprises a safety connection configured to connect the adjustable safety valve.

Preferably, the flow control and conditioning equipment comprises a pressure tap connection configured to connect the pressure transmitter. Preferably, said pressure tap connection is a male connection for connecting a 6 mm outer diameter oxygen line to a pressure tap of the pressure transmitter arranged on the control panel.

Preferably, the flow control and conditioning equipment comprises a nozzle configured to increase the kinetic energy of the continuous flow to increase the pressure value at the end of the intrinsic PEEP expiration.

Preferably, the nozzle is arranged internally within the flow control and conditioning equipment between the inspiratory inlet port and the endotracheal outlet port, opposite the expiratory outlet port.

Thus, gas injection into the system is done through a nozzle and in front of the expiratory outlet. The geometrical characteristics of this flow distribution zone actively contribute to obtain the desired clinical advantages. At the beginning of the expiratory phase, the airway pressure is high with respect to the external pressure, which tends to generate an expiratory flow. This flow is in the opposite direction to the air inflow into the system. This flow opposition generates a pneumatic resistance that confers the two characteristic clinical advantages of the system: slowing of expiration and maintenance of a final pressure (PEEP) different from atmospheric pressure.

The calibration of PEEP pressure and expiratory flow slowdown are obtained by sizing the inlet gas flow velocity and its position relative to the expiratory outlet. The inlet gas flow is a clinically useful parameter, whereby the critical inlet gas velocity is sized by the nozzle diameter at the injection point.

On the one hand, increasing the inlet gas velocity leads to higher pneumatic resistance and thus higher PEEP pressure. Likewise, the resistance of expiratory gas evacuation depends on the position of the injection point of the inlet flow with respect to the expiratory pathway. In particular, the closer the inlet gas injection point is to the endotracheal tube, the more it opposes the evacuation of exhaled gas.

To ensure the directionality of the gas flow at the nozzle exit and thus the desired expiratory pneumatic resistance, it is essential that the angle of the cone is appropriate. Too large an angle will generate a diffuse flow and the advantage of the system will be lost. A very weak angle will circulate the incoming gas flow in a long area of reduced cross-section, thus increasing the pneumatic resistance upstream of the nozzle and limiting the maximum flow that can pass through the system and consequently limiting the clinical use of the system.

Thus, key parameters for the correct functioning of the nozzle are: the relative position of the inlet gas injection with respect to the expiratory pathway, the diameter of the inlet gas injection nozzle, and the angle of the nozzle cone.

Preferably, the nozzle presents a conical configuration. Preferably, the nozzle presents an outlet orifice diameter of 0.5 mm to 1.5 mm. Preferably, the nozzle has an outlet orifice diameter of 1.2 mm. Preferably, the nozzle presents an inner angle or angle of attack of 13° to 15°.

The operating method, or ventilatory mode, of the ventilator for mechanical ventilation comprises at least the following steps:
- receive air and oxygen; and
- conditioning a continuous flow of such air and oxygen with controlled acceleration.

The ventilator for mechanical ventilation thus receives air and oxygen, produces a continuous flow of such air and oxygen, and accelerates it in a controlled manner to deliver an accelerated continuous flow of such air and oxygen.

The operating method of a ventilator for mechanical ventilation further comprises the following step:
- produce a collision of the accelerated continuous flow with a patient's expiratory flow, causing an expiratory brake and an intrinsic PEEP end-expiratory pressure.

The accelerated continuous flow has an opposite direction to the expiratory flow, so that when both flows collide, an intrinsic opposition to expiration is generated, accentuated by an acceleration of the fluid (gas mixture).

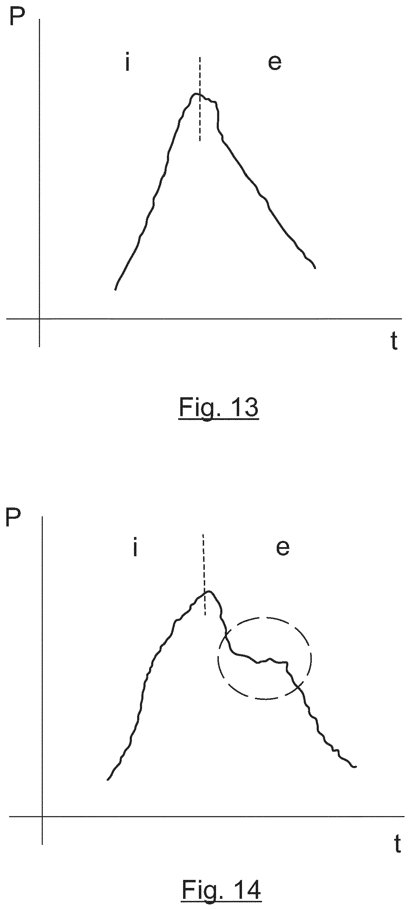

In other words, an intrinsic pressure opposite to expiration is provoked, which stabilizes the pressure decrease over time (increasing the mean expiratory pressure) in the expiratory phase, as shown in Figure 14.

The continuous flow remains constant during the patient's inspiratory and expiratory phases. The acceleration of the flow is controlled by the diameter of the outlet orifice and the angle of attack of the nozzle at a given point on the flow control and conditioning equipment. Specifically, with the nozzle outlet arranged opposite the expiratory outlet port that delivers fluid to the patient.

Preferably, the continuous flow collision with the expiratory flow coming from the patient is produced by opening an expiratory valve arranged in the expiratory branch, while keeping the continuous flow constant.

Preferably, the operating method comprises the following step:
- cause an added end-expiratory pressure PEEP.

Preferably, end-expiratory PEEP pressure is produced by regulating a PEEP valve arranged in series with the expiratory valve on the expiratory limb, configured to trigger, which can provide an added PEEP of between 0 to 22 cm H₂O. The added PEEP is in addition to the intrinsic PEEP.

Preferably, the operating method comprises the following step:
- increase the kinetic energy of the continuous flow (Fc) in a controlled manner to create turbulent flow.

This improves the overcoming of airway resistance and makes it possible to overcome alveolar compliance (ratio between the volume administered in an insufflation and the pressure exerted on the lung to introduce it).

Thus, the mode of operation described is based on a time-controlled mechanical ventilation technique characterized by a continuous gas flow, differentiated by the acceleration at a specific point applied at the entrance of the endotracheal tube by a single circuit (inspiratory and expiratory), producing the following effects:
- Accelerated fresh gas velocity, increasing the kinetic energy delivered and improving the overcoming of airway resistance.
- Highly turbulent flow of the fluid supplied, which allows overcoming the alveolar compliance produced by the increase in surface tension due to alveolar exudates and the loss of surfactant.
- Intrinsic expiratory brake effect in the system, increased by the acceleration of the fluid that collides with expiration in the expiratory phase. This maintains a mean expiratory pressure that prevents collapse of the alveoli in the declining zones of the lung.

The operating method, or ventilatory mode, of a ventilator for mechanical ventilation thus results in an increase in the mean expiratory pressure.

### BRIEF DESCRIPTION OF DRAWINGS

The following is a very brief description of a series of drawings which help to better understand the invention, and which relate expressly to two embodiments of said invention which are presented as non-limiting examples of the invention.
Figure 1 represents a pneumatic schematic of the ventilator of the present invention.
Figure 2 depicts a perspective view of the flow control and conditioning equipment of the ventilator of Figure 1.
Figure 3 represents a side view of the flow control and conditioning equipment of Figure 2.
Figure 4 represents a top view of the flow control and conditioning equipment of Figure 2.
Figure 5 represents a bottom view of the flow control and conditioning equipment of Figure 2.
Figure 6 represents a front view of the flow control and conditioning equipment of Figure 2.
Figure 7 represents a dorsal view of the flow control and conditioning equipment of Figure 2.
Figure 8 represents a longitudinal section of the flow control and conditioning equipment according to the cut line A-A of Figure 3, according to a first preferred embodiment.
Figure 9 represents a longitudinal section of the flow control and conditioning equipment according to the cut line A-A of Figure 3, according to a second preferred embodiment.
Figure 10 represents a longitudinal section of the flow control and conditioning equipment according to the cut line B-B of Figure 4, according to the first preferred embodiment.
Figure 11 represents a longitudinal section of the flow control and conditioning equipment according to the cut line B-B of Figure 4, according to the second preferred embodiment.
Figure 12 depicts a front view of the ventilator control box of Figure 1.
Figure 13 shows a graph of the pressure-time curve corresponding to a respiratory cycle, according to a conventional ventilatory mode.
Figure 14 shows a graph of the pressure-time curve corresponding to a respiratory cycle, according to the ventilatory mode of the ventilator of the present invention.

### DETAILED DESCRIPTION OF EMBODIMENTS

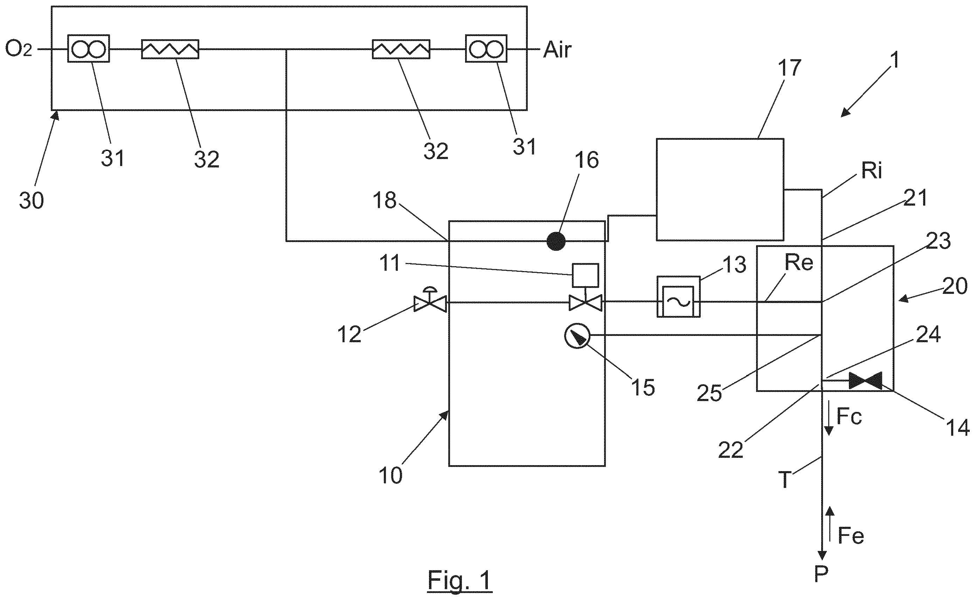

Figure 1 shows a pneumatic diagram of the continuous flow ventilator (1) for mechanical ventilation of the present invention. As can be seen, the ventilator (1) comprises a control box (10), an inspiratory limb (Ri) configured to receive an air-oxygen mixture, an expiratory limb (Re), and an expiratory valve (11) disposed on the expiratory limb (Re).

The ventilator (1) comprises a flow control and conditioning equipment (20) to which the inspiratory limb (Ri) and the expiratory limb (Re) are connected, configured to deliver a continuous flow (Fc) of said air-oxygen mixture to a patient (P) through an endotracheal tube (T), where said continuous flow (Fc) is kept constant during inspiration and expiration of the patient, so that upon opening of the expiratory valve (11) there is a collision of the continuous flow (Fc) with the expiratory flow (Fe) coming from the patient (P), causing an expiratory brake and an intrinsic PEEP end-expiratory pressure.

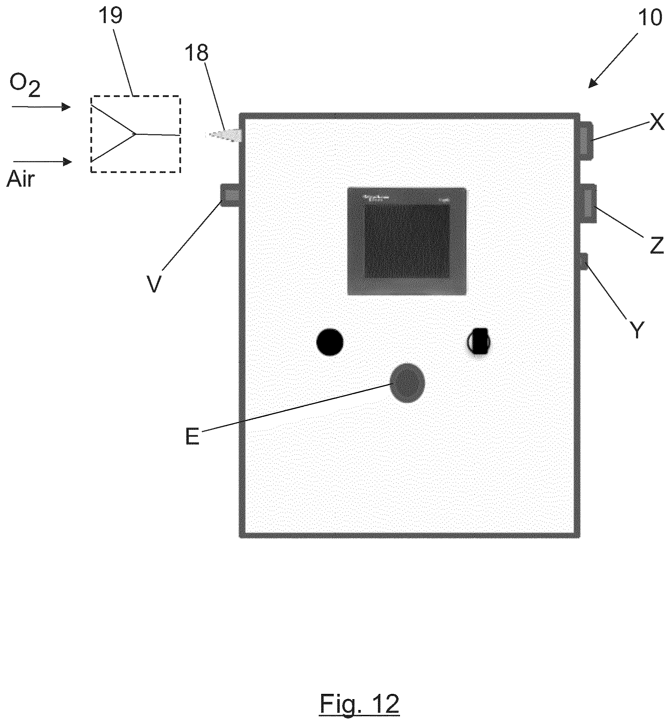

The ventilator (1) comprises a PEEP valve (12) arranged in series with the expiratory valve (11) on the expiratory branch (Re), configured to cause an added PEEP end-expiratory pressure. The PEEP valve (12) is positioned downstream of the expiratory valve (11) on an added pressure tap (V) arranged in the control box (10), Figure 12.

The ventilator (1) comprises an antibacterial and/or antiviral filter (13) arranged in series with the expiratory valve (11) in the expiratory branch (Re).

The ventilator (1) comprises an adjustable safety valve (14) connected to the flow control and conditioning equipment (20) configured to limit the inspiratory limb pressure (Ri).

The ventilator (1) comprises a pressure transmitter (15) connected to the flow control and conditioning equipment (20) configured to monitor the pressure during the respiratory cycle.

The ventilator (1) comprises an oxygen sensor (16) in the inspiratory limb (Ri) configured to measure the inspired oxygen fraction FiO₂.

The ventilator (1) comprises a humidifier (17) active in the inspiratory limb (Ri), configured to humidify the air-oxygen mixture.

The ventilator (1) comprises a gas inlet port (18) configured to receive the mixture of air and oxygen coming from a mixer (30).

The mixer (30) comprises a flow meter (31) and a humidifier (32) for both compressed air and oxygen.

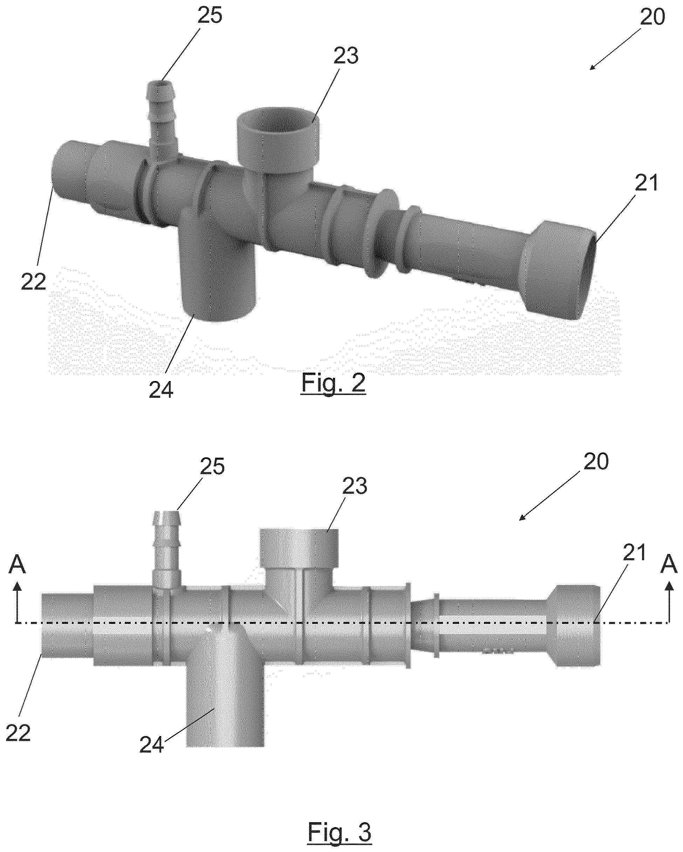

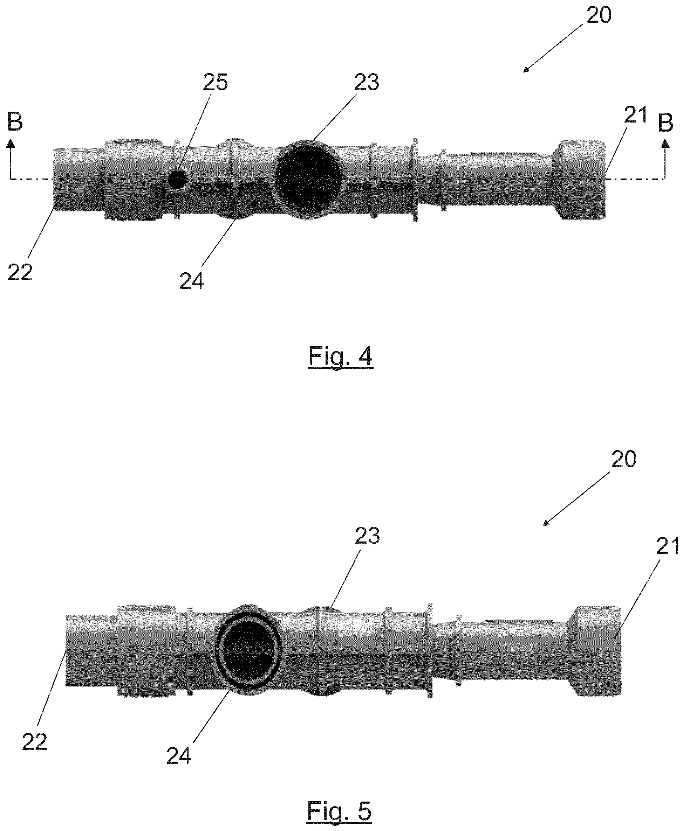

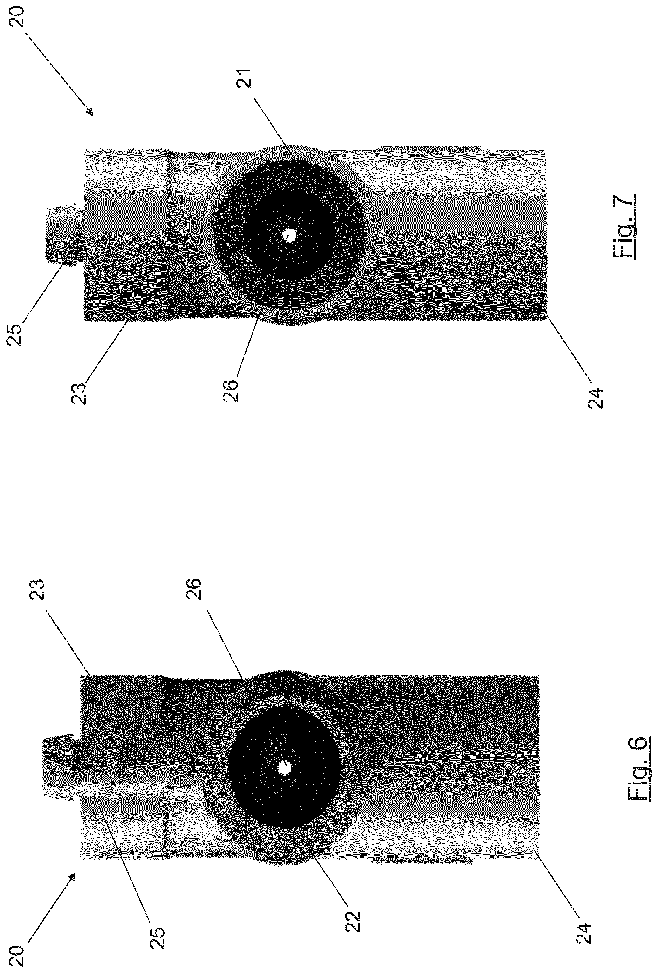

Figures 2 to 7 show various views of the flow control and conditioning equipment (20). As can be seen, it presents a tubular configuration comprising a plurality of pneumatically connected connections (21, 22, 23, 24, 25).

The flow control and conditioning equipment (20) comprises:
- an inspiratory inlet port (21) configured to connect to the inspiratory branch (Ri) and receive the air/oxygen mixture;
- an endotracheal outlet port (22), aligned with the inspiratory inlet port (21), configured to connect to the endotracheal tube (T) and deliver a continuous flow (Fc) of the air-oxygen mixture to the patient (P); and
- an expiratory outlet port (23), arranged in a "T" between the inspiratory inlet port (21) and the endotracheal outlet port (22), configured to connect to the expiratory branch (Re) and allow the expiratory flow (Fe) from the patient to exit (P).

The flow control and conditioning equipment (20) comprises a safety connection (24) configured to connect the adjustable safety valve (14).

The flow control and conditioning equipment (20) comprises a pressure tap connection (25) configured to connect the pressure transmitter (15).

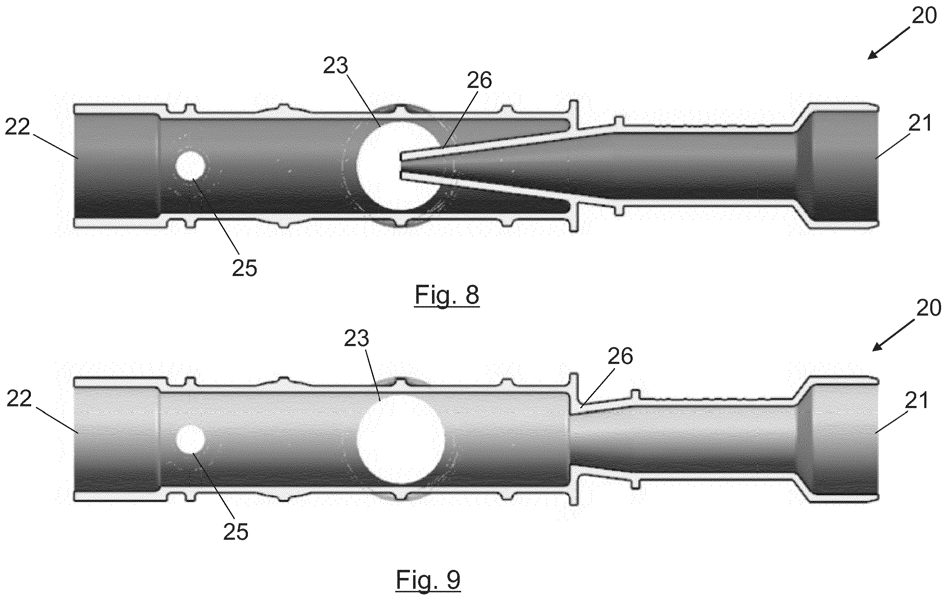

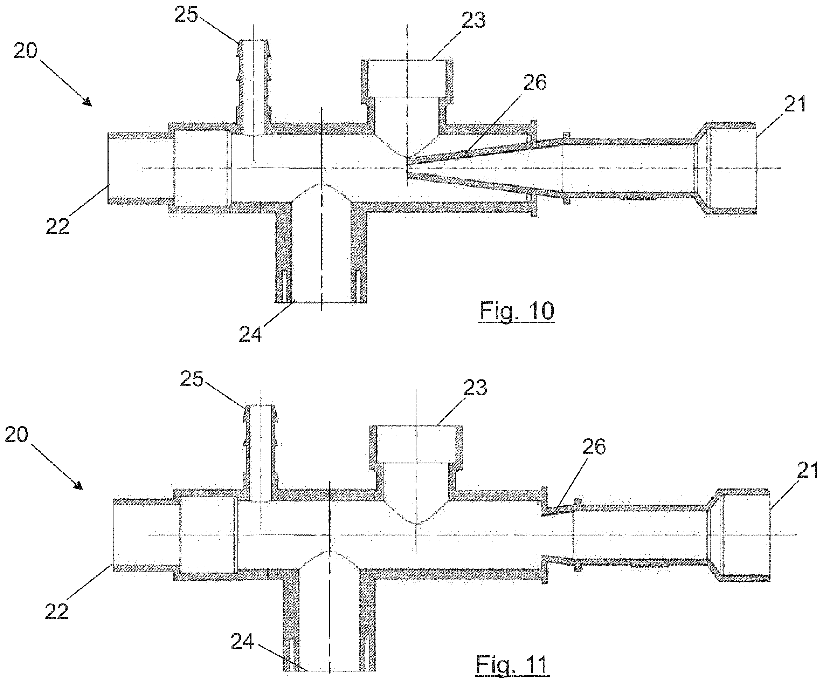

Figures 8 and 10 show two longitudinal sections of the flow control and conditioning equipment (20), according to a first preferred embodiment. This first configuration of the flow control and conditioning equipment (20), referred to as ECAF PRO, produces a system intrinsic PEEP of between 6 and 11 cm H2O for flow rates between 20 I/min and 30 I/min respectively.

For this purpose, the flow control and conditioning equipment (20) comprises a nozzle (26) configured to increase the kinetic energy of the continuous flow (Fc) to increase the pressure value at the end of the intrinsic PEEP expiration.

The nozzle (26) is arranged internally within the flow control and conditioning device (20) between the inspiratory inlet port (21) and the endotracheal outlet port (22), with the outlet of the nozzle (26) facing the expiratory outlet port (23).

The nozzle (26) has a conical configuration, with an outlet diameter of 0.5 mm to 1.5 mm, and an inner angle of 13° to 15°.

Figures 9 and 11 show two longitudinal sections of the flow control and conditioning equipment (20), according to a second preferred embodiment. This second configuration, referred to as ECAF STANDARD, produces a system intrinsic PEEP of between 2 and 3 cm H₂O for flow rates of between 20 I/min and 30 I/min respectively.

Figure 12 shows a front view of the control box (10). As can be seen, the gas inlet port (18) may additionally comprise a "Y" upstream connection (19) for separate connection of air and oxygen.

The control panel (10) comprises an inspiratory outlet (X) for connection of a corrugated tube coming from the humidifier (17) or coming from the inspiratory inlet connection (21) in case the humidifier (17) is not available.

The control panel (10) comprises an expiratory inlet (Z) for the connection of a corrugated tube coming from the expiratory outlet connection (23).

The control box (10) comprises a pressure tap (Y) of the pressure transmitter (15) for connecting an oxygen line coming from the pressure tap connection (25).

The control panel (10) comprises an additional pressure port (V) for connection of the PEEP valve (12).

The front of the control box (10) comprises a manual emergency push button (E) that allows manual opening of the expiratory valve (11) for patient safety.

Figure 13 shows a graph of the pressure-time curve corresponding to a respiratory cycle, according to a conventional ventilatory mode. The "I" represents the inspiration phase of the curve, while the "e" represents the expiration phase of the curve.

Figure 14 shows a graph of the pressure-time curve corresponding to a respiratory cycle according to the ventilatory mode of the ventilator of the present invention. The "i" represents the inspiration phase of the curve, while the "e" represents the expiration phase of the curve.

As can be seen, the collision of the continuous flow (Fc) with the expiratory flow (Fe) coming from the patient (P) causes an expiratory brake and an intrinsic end-expiratory pressure PEEP, as shown in the section of the curve inside the circle. This causes the mean expiratory pressure to be higher, compared to that shown in the curve in Figure 13.

## Claims

1. Ventilator for mechanical ventilation, comprising an inspiratory branch (Ri) configured to receive an air-oxygen mixture, an expiratory branch (Re), and an expiratory valve (11) arranged in the expiratory branch (Re), wherein said ventilator (1) comprises a flow control and conditioning equipment (20) to which the inspiratory branch (Ri) and the expiratory branch (Re) are connected, configured to supply a continuous flow (Fc) of said air-oxygen mixture to a patient (P), where said continuous flow (Fc) is kept constant during inspiration and expiration of the patient (P), so that when the expiratory valve (11) opens there is a collision of the continuous flow (Fc) with the expiratory flow (Fe) coming from the patient (P), causing an expiratory brake and an intrinsic PEEP end-expiratory pressure; **characterized in that** the flow control and conditioning equipment (20) presents a tubular configuration comprising a plurality of connections (21, 22, 23, 24, 25) communicated pneumatically; and **in that** said flow control and conditioning equipment (20) comprises:
- an inspiratory inlet port (21) configured to connect to the inspiratory branch (Ri) and receive the air/oxygen mixture;
- an endotracheal outlet port (22), aligned with the inspiratory inlet port (21), configured to connect to an endotracheal tube (T) of a patient (P) and deliver a continuous flow (Fc) of the air-oxygen mixture to said patient (P); and
- an expiratory outlet port (23), arranged in a "T" between the inspiratory inlet port (21) and the endotracheal outlet port (22), configured to connect to the expiratory branch (Re) and allow the expiratory flow (Fe) to exit from the patient (P).

2. Ventilator according to claim 1, **characterized in that** it comprises a PEEP valve (12) arranged in series with the expiratory valve (11) in the expiratory branch (Re), configured to cause an added PEEP end-expiratory pressure.

3. Ventilator according to any one of claims 1 to 2, **characterized in that** it comprises an antibacterial and/or antiviral filter (13) arranged in series with the expiratory valve (11) in the expiratory branch (Re).

4. Ventilator according to any one of claims 1 to 3, **characterized in that** it comprises an adjustable safety valve (14) connected to the flow control and conditioning equipment (20) configured to limit the inspiratory branch pressure (Ri).

5. Ventilator according to any one of claims 1 to 4, **characterized in that** it comprises a pressure transmitter (15) connected to the flow control and conditioning equipment (20) configured to monitor the pressure during the respiratory cycle.

6. Ventilator according to claim 4, **characterized in that** the flow control and conditioning equipment (20) comprises a safety connection (24) configured to connect the adjustable safety valve (14).

7. Ventilator according to claim 5, **characterized in that** the flow control and conditioning equipment (20) comprises a pressure tap connection (25) configured to connect the pressure transmitter (15).

8. Ventilator according to any one of claims 1 to 7, **characterized in that** the flow control and conditioning equipment (20) comprises a nozzle (26) configured to increase the kinetic energy of the continuous flow (Fc) to increase the value of the pressure at the end of intrinsic PEEP expiration.

9. Ventilator according to claim 8, **characterized in that** the nozzle (26) is arranged internally within the flow control and conditioning equipment (20) between the inspiratory inlet port (21) and the endotracheal outlet port (22).

10. Ventilator according to any one of claims 8 to 9, **characterized in that** the nozzle (26) has a conical configuration.

11. Ventilator according to any one of claims 8 to 10, **characterized in that** the nozzle (26) has an outlet orifice diameter of 0.5 mm to 1.5 mm.

12. Ventilator according to any one of claims 8 to 11, **characterized in that** the nozzle (26) has an outlet orifice diameter of 1.2 mm.

13. Ventilator according to any of the claims 8 to 12, **characterized in that** the nozzle (26) has an inner angle of 13° to 15°.

## Patentansprüche

1. Beatmungsgerät für mechanische Beatmung, umfassend einen Inspirationszweig (Ri), der zur Aufnahme eines Luft-Sauerstoff-Gemischs ausgelegt ist, einen Exspirationszweig (Re) und ein im Exspirationszweig (Re) angeordnetes Exspirationsventil (11), wobei das genannte Beatmungsgerät (1) eine Durchflussregelungs- und Konditionierungseinheit (20) in Verbindung mit dem Inspirationszweig (Ri) und dem Exspirationszweig (Re) umfasst, die dazu ausgelegt ist, einem Patienten (P) einen kontinuierlichen Strom (Fc) des genannten Luft-Sauerstoff-Gemischs zuzuführen, wobei der genannte kontinuierliche Strom (Fc) während der Inspiration und Exspiration des Patienten (P) konstant gehalten wird, sodass beim Öffnen des Exspirationsventils (11) eine Kollision des kontinuierlichen Stroms (Fc) mit dem vom Patienten (P) kommenden Exspirationsstrom (Fe) erfolgt, was einen Ausatmungswiderstand und einen intrinsischen endexspiratorischen Druck (PEEP) bewirkt; **dadurch gekennzeichnet, dass** die Durchflussregelungs- und Konditionierungseinheit (20) eine röhrenförmige Struktur mit einer Vielzahl von pneumatisch miteinander verbundenen Anschlüssen (21, 22, 23, 24, 25) aufweist; und dass die genannte Durchflussregelungs- und Konditionierungseinheit (20) Folgendes umfasst:
- eine Inspirationseinlassöffnung (21), die zum Anschluss an den Inspirationszweig (Ri) und zur Aufnahme des Luft-Sauerstoff-Gemisches ausgelegt ist;
- eine endotracheale Auslassöffnung (22), die auf die Inspirationseinlassöffnung (21) abgestimmt ist und zum Anschluss an einen Endotrachealtubus (T) eines Patienten (P) sowie zur Abgabe eines kontinuierlichen Stroms (Fc) des Luft-Sauerstoff-Gemischs an den genannten Patienten (P) ausgelegt ist; und
- eine Exspirationsauslassöffnung (23), die in T-Form zwischen der Inspirationseinlassöffnung (21) und der endotrachealen Auslassöffnung (22) angeordnet zum Anschluss an den Exspirationszweig (Re) ausgelegt ist und den Austritt des Exspirationsstroms (Fe) aus dem Patienten (P) ermöglicht.

2. Beatmungsgerät nach Anspruch 1, **dadurch gekennzeichnet, dass** es ein PEEP-Ventil (12) umfasst, das im Exspirationszweig (Re) in Reihe mit dem Exspirationsventil (11) angeordnet und dazu ausgelegt ist, einen zusätzlichen endexspiratorischen Druck (PEEP) zu erzeugen.

3. Beatmungsgerät nach einem der Ansprüche 1 bis 2, **dadurch gekennzeichnet, dass** es einen antibakteriellen und/oder antiviralen Filter (13) umfasst, der im Exspirationszweig (Re) in Reihe mit dem Exspirationsventil (11) angeordnet ist.

4. Beatmungsgerät nach einem der Ansprüche 1 bis 3, **dadurch gekennzeichnet, dass** es ein mit der Durchflussregelungs- und Konditionierungseinheit verbundenes einstellbares Sicherheitsventil (14) umfasst, das zur Begrenzung des Inspirationszweigdrucks (Ri) ausgelegt ist.

5. Beatmungsgerät nach einem der Ansprüche 1 bis 4, **dadurch gekennzeichnet, dass** es einen mit der Durchflussregelungs- und Konditionierungseinheit verbundenen Druckmessumformer (15) umfasst, der zur Überwachung des Drucks während des Respirationszyklus ausgelegt ist.

6. Beatmungsgerät nach Anspruch 4, **dadurch gekennzeichnet, dass** die Durchflussregelungs- und Konditionierungseinheit (20) einen Sicherheitsanschluss (24) umfasst, der zum Anschluss des einstellbaren Sicherheitsventils (14) ausgelegt ist.

7. Beatmungsgerät nach Anspruch 5, **dadurch gekennzeichnet, dass** die Durchflussregelungs- und Konditionierungseinheit (20) einen Druckabgriffanschluss (25) umfasst, der zum Anschluss des Druckmessumformers (15) ausgelegt ist.

8. Beatmungsgerät nach einem der Ansprüche 1 bis 7, **dadurch gekennzeichnet, dass** die Durchflussregelungs- und Konditionierungseinheit (20) eine Düse (26) umfasst, die dazu ausgelegt ist, die kinetische Energie des kontinuierlichen Stroms (Fc) zu erhöhen, um den Wert des intrinsischen endexspiratorischen Drucks (PEEP) zu steigern.

9. Beatmungsgerät nach Anspruch 8, **dadurch gekennzeichnet, dass** die Düse (26) im Inneren der Durchflussregelungs- und Konditionierungseinheit (20) zwischen der Inspirationseinlassöffnung (21) und der endotrachealen Auslassöffnung (22) angeordnet ist.

10. Beatmungsgerät nach einem der Ansprüche 8 bis 9, **dadurch gekennzeichnet, dass** die Düse (26) eine konische Form aufweist.

11. Beatmungsgerät nach einem der Ansprüche 8 bis 10, **dadurch gekennzeichnet, dass** die Düse (26) einen Auslassöffnungsdurchmesser von 0,5 mm bis 1,5 mm aufweist.

12. Beatmungsgerät nach einem der Ansprüche 8 bis 11, **dadurch gekennzeichnet, dass** die Düse (26) einen Auslassöffnungsdurchmesser von 1,2 mm aufweist.

13. Beatmungsgerät nach einem der Ansprüche 8 bis 12, **dadurch gekennzeichnet, dass** die Düse (26) einen Innenwinkel von 13° bis 15° aufweist.

## Revendications

1. Respirateur pour ventilation mécanique, comprenant une branche inspiratoire (Ri) configurée pour recevoir un mélange d'air-oxygène, une branche expiratoire (Re), et une valve expiratoire (11) disposée dans la branche expiratoire (Re), dans lequel ledit respirateur (1) comprend un équipement de commande et de conditionnement de flux (20) auquel sont reliées la branche inspiratoire (Ri) et la branche expiratoire (Re), configuré pour fournir un flux continu (Fc) dudit mélange d'air-oxygène au patient (P), dans lequel ledit flux continu (Fc) est maintenu constant pendant l'inspiration et l'expiration du patient (P), de telle sorte que lorsque la valve expiratoire (11) s'ouvre il y a une collision du flux continu (Fc) avec le flux expiratoire (Fe) provenant du patient (P), provoquant un frein expiratoire et une pression de fin d'expiration PEEP intrinsèque; **caractérisé en ce que** l'équipement de commande et de conditionnement de flux (20) présente une configuration tubulaire comprenant une pluralité de connexions (21, 22, 23, 24, 25) communiquées pneumatiquement; et **en ce que** ledit équipement de commande et de conditionnement de flux (20) comprend:
- un orifice d'entrée inspiratoire (21) configuré pour se connecter à la branche inspiratoire (Ri) et recevoir le mélange d'air/oxygène ;
- un orifice de sortie endotrachéal (22), aligné avec l'orifice d'entrée inspiratoire (21), configuré pour se connecter à un tube endotrachéal (T) d'un patient (P) et délivrer un flux continu (Fc) du mélange d'air-oxygène audit patient (P); et
- un orifice de sortie expiratoire (23), disposé en « T » entre l'orifice d'entrée inspiratoire (21) et l'orifice de sortie endotrachéal (22), configuré pour se connecter à la branche expiratoire (Re) et permettre au flux expiratoire (Fe) de sortir du patient (P).

2. Respirateur selon la revendication 1, **caractérisé en ce qu'il** comprend une valve de PEEP (12) disposée en série avec la valve expiratoire (11) dans la branche expiratoire (Re), configurée pour provoquer une pression de fin d'expiration PEEP ajoutée.

3. Respirateur selon l'une quelconque des revendications 1 à 2, **caractérisé en ce qu'**il comprend un filtre antibactérien et/ou antiviral (13) disposé en série avec la valve expiratoire (11) dans la branche expiratoire (Re).

4. Respirateur selon l'une quelconque des revendications 1 à 3, **caractérisé en ce qu'**il comprend une valve de sécurité réglable (14) reliée à l'équipement de commande et de conditionnement de flux (20) configurée pour limiter la pression de la branche inspiratoire (Ri).

5. Respirateur selon l'une quelconque des revendications 1 à 4, **caractérisé en ce qu'**il comprend un transmetteur de pression (15) relié à l'équipement de commande et de conditionnement de flux (20) configuré pour surveiller la pression pendant le cycle respiratoire.

6. Respirateur selon la revendication 4, **caractérisé en ce que** l'équipement de commande et de conditionnement de flux (20) comprend une connexion de sécurité (24) configurée pour connecter la valve de sécurité réglable (14).

7. Respirateur selon la revendication 5, **caractérisé en ce que** l'équipement de commande et de conditionnement de flux (20) comprend une connexion de prise de pression (25) configurée pour connecter le transmetteur de pression (15).

8. Respirateur selon l'une quelconque des revendications 1 à 7, **caractérisé en ce que** l'équipement de commande et de conditionnement de flux (20) comprend une buse (26) configurée pour augmenter l'énergie cinétique du flux continu (Fc) pour augmenter la valeur de la pression à la fin de l'expiration PEEP intrinsèque.

9. Respirateur selon la revendication 8, **caractérisé en ce que** la buse (26) est disposée à l'intérieur de l'équipement de commande et de conditionnement de flux (20) entre l'orifice d'entrée inspiratoire (21) et l'orifice de sortie endotrachéal (22).

10. Respirateur selon l'une quelconque des revendications 8 à 9, **caractérisé en ce que** la buse (26) présente une configuration conique.

11. Respirateur selon l'une quelconque des revendications 8 à 10, **caractérisé en ce que** la buse (26) présente un diamètre d'orifice de sortie de 0,5 mm à 1,5 mm.

12. Respirateur selon l'une quelconque des revendications 8 à 11, **caractérisé en ce que** la buse (26) présente un diamètre d'orifice de sortie de 1,2 mm.

13. Respirateur selon l'une quelconque des revendications 8 à 12, **caractérisé en ce que** la buse (26) présente un angle interne de 13° à 15°.
